# EUROPEAN PATENT SPECIFICATION

(11) **EP 2 653 100 B1**
(45) Date of publication and mention of the grant of the patent: **22.07.2015**
(21) Application number: 11848626.5
(22) Date of filing: 20.09.2011
(51) Int. Cl.: A61B 5/151

(54) **LANCET DEVICE CAPABLE OF ADJUSTING SUBCUTANEOUS PENERATION DEPTH**
LANZETTE MIT EINSTELLUNG DER SUBKUTANEN PENERATIONSTIEFE
DISPOSITIF À LANCETTE PERMETTANT DE RÉGLER LA PROFONDEUR DE PÉNÉTRATION SOUS-CUTANÉE

(30) Priority: 15.12.2010 KR 20100128558
(43) Date of publication of application: 23.10.2013
(73) Proprietor: i-Sens, Inc., Nowon-gu, Seoul 139-050 (KR)
(72) Inventor: CHA, Geun Sig, Seoul 120-100 (KR); NAM, Hakhyun, Seoul 142-063 (KR); CHA, Eun-Jong, Cheongju-si Chungcheongbuk-do 361-753 (KR); KIM, Kyung-Ah, Cheongju-si Chungcheongbuk-do 361-765 (KR)
(74) Representative: Modiano, Micaela Nadia
(86) International application number: PCT/KR2011/006938
(87) International publication number: WO 2012/081810

(56) References cited:
- EP-A1- 1 625 824
- WO-A2-2009/136171
- JP-B2- 4 302 517
- KR-A- 19990 082 310
- KR-A- 20090 049 505
- KR-A- 20100 120 403
- US-A1- 2004 249 406
- US-A1- 2005 159 768

## Description

### Field of the Invention

The present invention relates to a lancet device with an adjustable hypodermic penetration depth. More particularly, the present invention relates to a lancet device that can minutely adjust the hypodermic penetration depth of a lancet needle in accordance with the subject or the blood-taking portion.

### Description of the Related Art

In general, chronic diabetics have to measure the blood glucose level by performing a blood sugar test by themselves everyday at home and to perform disease control in order to keep a predetermined blood glucose level.

They have to collect blood for the blood glucose test, and this case, generally, they stick a disposable lancet into the skin of a portion, usually a finger, of their bodies, take and put a small amount of capillary blood onto a strip, and then measure the blood glucose level using a blood glucose meter with the strip mounted.

A lancing device is generally used as the device for taking blood.

The lancing device is composed of a lancet holder mounted with a disposable lancet, a cover that covers a lancet and has a hole through which only the tip of a needle protrudes to penetrate a skin, and a spring and a releasing member that provide a penetration force. The disposable lancet has a lancet needle at one end of a lancet body and a protection cap is combined with the lancet needle,

According to the lancing devices having this configuration in the related art, a user removes the cover from a lancing device, mounts a disposable lancet onto the lancet holder, attaches the cover with the spring compressed, brings the lancet in close contact with a portion with many capillaries such as fingers, and then releases the disposable lancet by pulling a releasing switch, such that the lancet penetrates the skin.

The lancet needle is fixed in length that penetrates a skin is fixed, the thickness of the skin of people is different and the portion from which blood is taken may be different, such that it is required to make the length of the lancet needle penetrating the skin different in accordance with the subject or the blood-taking portion.

Further, inflammation may be caused, when the lancet needle penetrates a skin unexpectedly deep, such that it is required to minutely adjust the hypodermic penetration depth of the lancet needle.

Examples of lancet devices with lancing depth adjustment are known from the following documents.

US 2005/0159768 A1 discloses an apparatus and method for lancing a surface provides an adjustable nozzle assembly that includes an interior nozzle, a collar, and an exterior nozzle with a surface that contacts the surface to be lance The exterior nozzle can then rotate relative to the interior nozzle and, thereby, vary the lancing depth of a lancet.

US 2004/0249406 A1 discloses a lancing device having a low-mass, high-velocity lancet decoupled from a drive mechanism when piercing a user's skin. The drive mechanism includes a spring-driven piston that impacts the lancet on firing to propel the lancet from a retracted position towards an extended position. After impact, but before the lancet reaches the extended position, a stop limits the travel of the piston to separate the piston from the lancet. Precise guidance of the lancet minimizes lateral movement of the lancet.

EP 1 625 824 discloses a lancing depth-adjustable lancing apparatus for moving a lancing element held in a housing in a lancing direction to lance skin with the lancing element. The lancing apparatus includes a lancing depth adjustment mechanism for adjusting lancing depth of the lancing element in the skin. The lancing depth adjustment mechanism includes a displacing member which is movable relative to the housing in the lancing or the retreating direction, a cover which is movable with the displacing member in the lancing or the retreating directions, and a stepped portion provided at the housing for restricting the movement of the lancing element in the lancing direction when the lancing element moves in the lancing direction.

### Disclosure

### Technical Problem

The present invention has been made in an effort to provide a lancet device having advantages of being able to minutely adjust the hypodermic penetration depth of a lancet needle in accordance with the subject or the blood-taking portion.

### Technical Solution

An exemplary embodiment of the present invention provides a lancet device with an adjustable hypodermic penetration depth, which includes: a lancet holder having a lancet seat to mount a lancet at one end; an operating mechanism disposed at the other end of the lancet holder and loading and releasing the lancet holder along a predetermined path; a depth adjusting member receiving the lancet holder and having at least two grooves formed at a predetermined angle on the circumferential surface of one end to form inclined passages clockwise or counterclockwise; and a housing receiving the lancet holder and the operating mechanism and having a protrusion that is formed on the inner side of an end and fitted step by step in the grooves when the depth adjusting member turns at a predetermined angle.

As described above, according to an exemplary embodiment of the present invention, a subject can conveniently adjust the hypodermic penetration depth of the lancet needle by turning the cover or the housing step by step, and subjects can minutely adjust the hypodermic penetration depth of the lancet needle in accordance with the thickness of their skin or the blood-taking portion.

### Description of Drawings

FIG. 1 is a perspective view of a lancet device with an adjustable hypodermic penetration depth according to an exemplary embodiment of the present invention.
FIG. 2 is an exploded perspective view of FIG. 1.
FIG. 3 is a cross sectional side view of FIG. 1.
FIG. 4 is an enlarged perspective view showing a depth adjusting member and a protrusion of FIG. 2 to examine a method of adjusting a hypodermic penetration depth.
FIG. 5 is a schematic view showing the depth adjusting member and the protrusions in a plane to examine the method of adjusting a hypodermic penetration depth.

### Best Mode

Hereinafter, exemplary embodiments of the present invention will be described in detail with reference to the accompanying drawings. First, when giving the components in the drawings reference numerals, it should be noted that the same reference numerals are given to reference numerals as same as possible even if they are shown in different drawings. Further, the in description of the present invention, the detailed description of related well-known configurations and functions is not provided, when it is determined as making the scope of the present invention unclear.

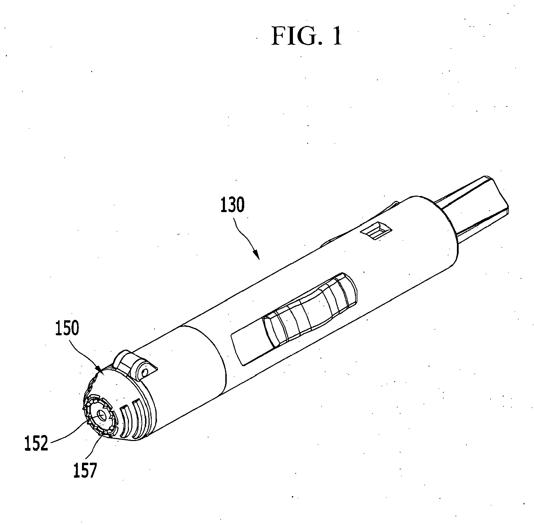

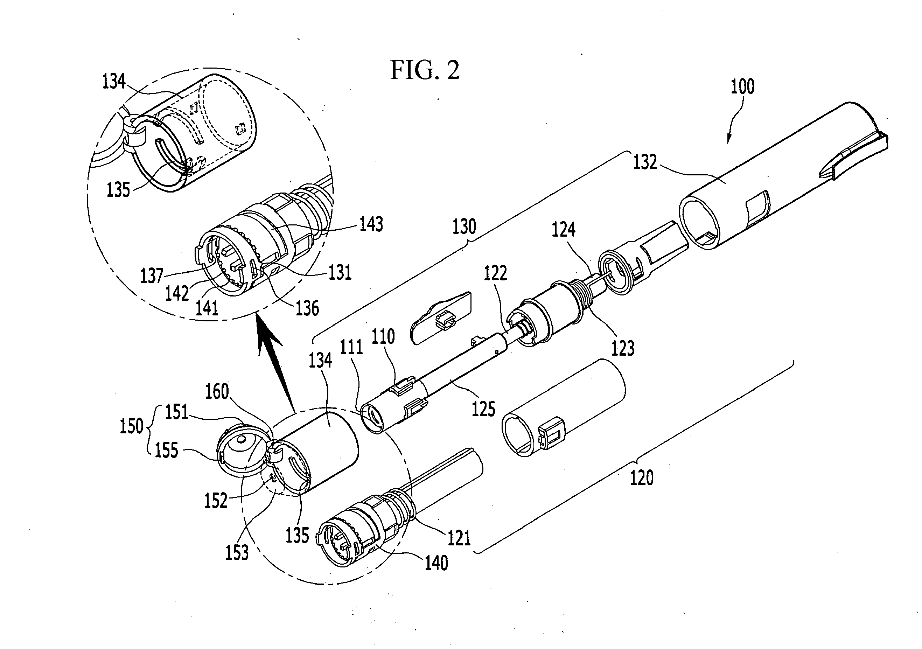

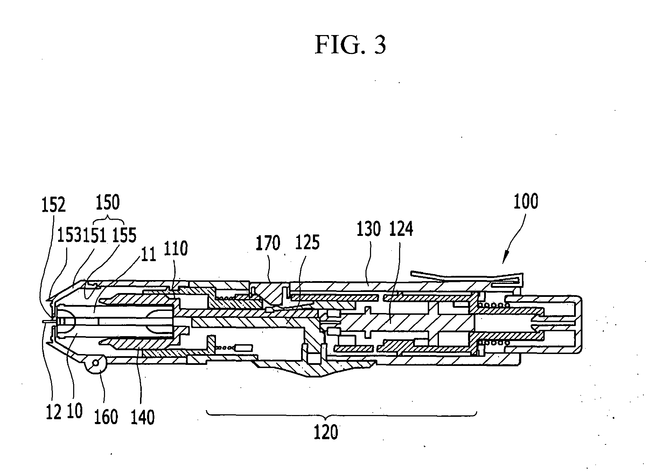

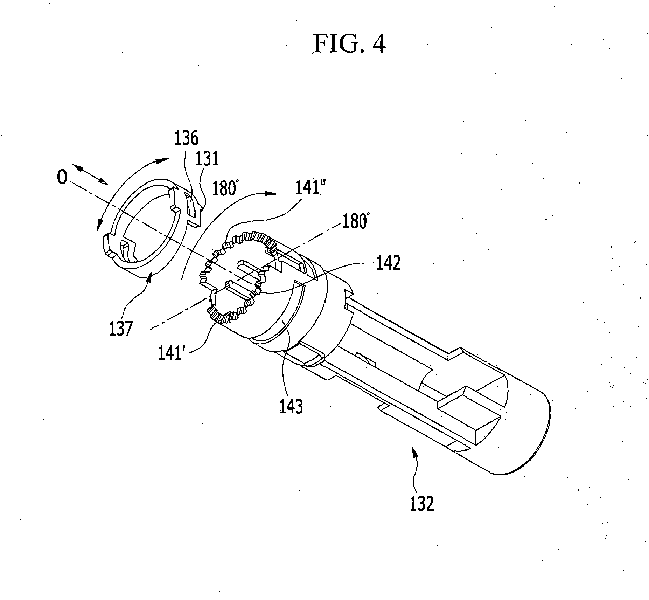

FIG. 1 is a perspective view of a lancet device with an adjustable hypodermic penetration depth according to an exemplary embodiment of the present invention, FIG. 2 is an exploded perspective view of FIG. 1, FIG. 3 is a cross sectional side view of FIG. 1, and FIG. 4 is an enlarged perspective view showing a depth adjusting member and a protrusion of FIG. 2 to examine a method of adjusting a hypodermic penetration depth.

Referring to FIGS. 1 to 4, a lancet device 100 with an adjustable hypodermic penetration depth according to an exemplary embodiment of the present invention largely includes a lancet holder 110, an operating mechanism 120, an operation switch 170, a housing 130, a depth adjusting member 140, a cover 150, and a coupling portion 160.

The lancet holder 110 has a lancet seat 111 to mount a lancet 10 at one end and is disposed at a side in the housing 130.

The lancet 10, which is disposable, has a lancet needle 12 at one end of a lancet body 11 with a predetermined length and the lancet needle 12 is combined with a protection cap (not shown).

The protection cap is coupled to one end of the lancet needle 12 by insert injection, and according to this configuration, the protection cap can be separated from the lancet needle 12 even by a small force.

The operating mechanism 120 is disposed at the other end of the lancet holder 110 and has a structure for loading and releasing the lancet holder 110 along a predetermined path, and for this operation, it includes a plurality of springs 121, 122, and 123, as shown in FIG. 1.

The operation switch 170 is connected to the operating mechanism 120 and operates the operating mechanism 120 by being pressed.

The depth adjusting member 140 receives the lancet holder 110 and has at least two grooves 142 formed at a predetermined angle along two inclined passages 141 on the circumferential surface of one end.

In an exemplary embodiment of the present invention, the inclined passages 141 are formed symmetrically with a gap of 180 degrees. In other words, the inclined passages 141 have a first inclined passage 141' with the inclination decreasing clockwise from 0°to 180°and a second inclined passage 141" with the inclination increasing counterclockwise from 0°to 180°, and a step 145 having a predetermined height is formed at the joint of the first inclined passage 141' and the second inclined passage 141" so that the depth adjusting member 140 is controlled not to turn 360 degrees.

Such a configuration will be described in detail below.

The housing 130, a cylinder sized to be carried by a user, receives the lancet holder 110 and the operating mechanism 120 and has a protrusion 131 that is formed on the inner side of an end and fitted step by step in the grooves 142 when turning at a predetermined angle, such that the distance I between the lancet needle 12 and the through-hole 151 is adjusted.

Further, the housing 130 includes a first housing part 132 receiving the operating mechanism 120 and a second housing part 134 rotating about the axis of the first housing part 132, combined with the depth adjusting member 140 therein, and having the protrusion 131.

The cover 150 is hinged to the second housing part 134 and has a through-hole 151 at the center through which a lancet needle 12 of the lancet passes.

The coupling portion 160 hinges the cover 150 to the second housing part 134, and according to this structure, it is possible to remove the problem in that a user has to separate the cover 150 from the lancet device 100 and then places it or hold it in a hand or the problem of losing the cover 150 while mounting and separating the lancet 10.

As described above, the inclined passage 141 formed around an end of the depth adjusting member 140 has the first inclined passage 141' with the inclination decreasing clockwise from 0° to 180° and the second inclined passage 141" with the inclination increasing counterclockwise from 0°to 180°, the step 145 having a predetermined height is formed at the joint of the first inclined passage 141' and the second inclined passage 141", and two protrusions 131 are symmetrically formed at a gap of 180 degrees to correspond to the grooves 142 formed on the inclined passage 141, such that clockwise or counterclockwise rotation is allowed only from 0° to 180°.

At least two guide protrusions 135 are formed in the same inclining direction as the inclined passage 141, on the inner side of the second housing part 134, for guiding combination and rotation between the second housing part 134 and the depth adjusting member 140 and a rotation guide groove 143 is formed at the depth adjusting member 140 along the movement path of the guide protrusions 135.

When the protrusion 131 rotates while moving to the grooves 142 along the inclined passage 141, the guide protrusions 135 guide the protrusion 131 moving while rotating in the same direction as the rotation described above along the slope of the rotation guide groove 143.

Simultaneously, the guide protrusions 135 and the rotation guide groove 143 bring the second housing part 134 and the depth adjusting member 140 in close contact with each other.

It is preferable that a hollow space 136 is defined above the protrusion 131 to allow the protrusion 131 to move to the grooves 142 along the inclined passage 141.

The hollow space 136 is provided to use the elastic force of plastic that is the material of the housing 130, and thus the protrusion 131 can minutely move up/down.

Therefore, the protrusion 131 can move to the grooves 142 from the inclined passage 141.

Further, noise is generated, when the protrusion moves along the grooves 142, such that a subject can estimate the degree of rotation of the protrusion 131 only from the noise.

The hypodermic penetration depth of the lancet needle 12 of the lancet 10 is determined within 1.7 to 2.0mm, which is achieved by adjusting the distance I between the lancet needle 12 and the through-hole 151.

The hypodermic penetration depth of the lancet needle 12, that is, the distance I between the lancet needle 12 and the through-hole 151 can be minutely adjusted from first to tenth steps, because ten grooves 142 can be formed in the inclined passage 141 in an exemplary embodiment of the present invention, and the higher the step, the more the distance I between the lancet needle 12 and the through-hole 151 decreases, such that the hypodermic penetration depth of the lancet needle 12 increases.

According to this configuration, a subject can conveniently adjust the hypodermic penetration depth of the lancet needle 12 by turning the cover 150 or the second housing part 134 step by step.

Further, subjects can minutely adjust the hypodermic penetration depth of the lancet needle 12 in accordance with the thickness of their skin or the blood-taking portion.

It is preferable that the step, which is set to let a subject know which step of the first step to the tenth step the hypodermic penetration depth of the lancet needle 12 is set to, in accordance with the position of the protrusion 131, is indicated partially on the surface of the cover 150 or the housing 130.

As described above, since the hypodermic penetration depth of the lancet needle 12 can be adjusted from first to tenth steps, ten grooves 142 are formed between 0 and 180 degrees so that the inclination can decrease at a gap of 18 degrees and ten grooves 142 are formed between 180 and 360 degrees too so that the inclination can decrease at a gap of 18 degrees.

Further, the depth adjusting member 140 can rotate clockwise or counterclockwise only from 0° to 180°, because the step 145 having a predetermined height is formed at the joint of the first inclined passage 141' and the second inclined passage 141 ".

As described above, since the depth adjusting member can rotate clockwise or counterclockwise only from 0° to 180°, it is possible to remove the problem in that the cover 150 is separated from the housing or the depth adjusting member 140 is damaged due to too much rotation, and to minutely adjust the hypodermic penetration depth.

Referring to FIG. 3, the operation order of the lancet device 100 is briefly described hereafter.

First, a subject sets the hypodermic penetration depth of the lancet needle 12 to a predetermined step in the first to tenth steps by turning the cover 150 or the second housing part 134, and when the subject presses the operation switch 170, the springs 121, 122, and 123 are compressed and then instantaneously extend down, such that a shock transmission rod 124 hits a movable shaft 125 positioned under the shock transmission rod.

The movable shaft 125 hits the lancet holder 110 with the lancet 10 fixed, such that the lancet needle 12 instantaneously protrudes outward and penetrates the skin of the subject at the predetermined hypodermic penetration depth according to the set step.

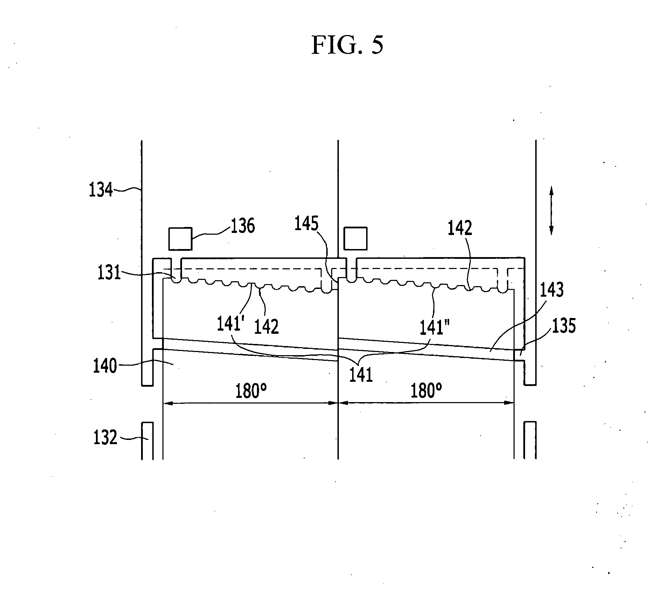

Next, a method of adjusting the hypodermic penetration depth of the lancet needle 12 is described in detail with reference to FIG. 5.

FIG. 5 is a schematic view showing the depth adjusting member and the protrusion in a plane to examine the method of adjusting a hypodermic penetration depth.

As shown in FIG. 4, as the cover 150 or the second housing part 134 is turned clockwise or counterclockwise within 0 to 180 degrees, the protrusion 131 is also turned in the same rotational direction as the cover 150 or the second housing part 134.

In this operation, the protrusion 131 moves to the grooves 142 along the inclined passage 141 formed on the depth adjusting member 140, as shown in FIG. 5, and by this movement, the cover 150 and the second housing part 134 are moved up/down together.

Therefore, the distance I (see FIG. 3) between the lancet needle 12 and the through-hole 151 of the cover is adjusted and the hypodermic penetration depth of the lancet needle 12 can be determined.

The above description is an example of the spirit of the present invention and may be changed and modified in various ways by those skilled in the art without departing from the scope of the present invention. Therefore, the exemplary embodiments described herein are not for limiting the spirit of the present invention, but for explaining the present invention and the scope of the present invention is not limited to the exemplary embodiments. The protective range of the present invention should be construed by claims and the equivalents should be construed as being included in the scope of the present invention.

While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims.

## Claims

1. A lancet device with an adjustable hypodermic penetration depth, comprising:
a lancet holder(110) having a lancet seat(111) to mount a lancet(10) at one end;
an operating mechanism(120) disposed at the other end of the lancet holder(110) and loading and releasing the lancet holder(110) along a predetermined path;
a depth adjusting member (140) receiving the lancet holder(110) and having at least two grooves (142) formed at a predetermined angle on the circumferential surface of one end to form inclined passages (141, 141'. 141") clockwise or counterclockwise; and
a housing (130) receiving the lancet holder (110) and the operating mechanism (120), **characterized in that**
said housing (130) has a protrusion (131) that is formed on the inner side of an end and is fitted step by step in the grooves (142) when the depth adjusting member (140) turns at a predetermined angle,
the housing (130) further including:
a first housing part (132) receiving the operating mechanism (120); and
a second housing part (134) rotating about the axis of the first housing part (132), combined with the depth adjusting member (140) therein, and having the protrusion (131),
the inclined passages (141) and the grooves (142) are formed symmetrically with a gap of 180 degrees,
two pieces of the protrusion (131) are formed symmetrically with a gap of 180 degrees and the hypodermic penetration depth of lancet needle of the lancet is adjusted from 1.7 to 2.0mm in ten steps, and
a hollow space (136) is defined above the protrusion (131).

2. The device of claim [1], wherein at least two guide protrusions(135) are formed in the same inclining direction as the inclined passage (141), on the inner side of the second housing part(134), for guiding combination and rotation between the second housing part (134) and the depth adjusting member(140), and a rotation guide groove (143) is formed at the depth adjusting member along the movement path of the guide protrusions (135).

3. The device of claim [1], further comprising a cover (150) hinged to the second housing part(135) and having a through-hole(151) at the center through which a lancet needle of the lancet passes.

4. The device of claim 1, wherein the inclined passages (141) have a first inclined passage with the inclination decreasing clockwise from 0°to 180° and a second inclined passage with the inclination increasing counterclockwise from 0° to 180°, and a step having a predetermined height is formed at the joint of the first inclined passage (141') and the second inclined passage (141").

5. The device of claim 4, wherein ten grooves (142) are formed at each of the first and second inclined passages(141', 141").

## Patentansprüche

1. Eine Lanzettenvorrichtung mit einer einstellbaren subkutanen Einbringtiefe, umfassend:
einen Lanzettenhalter (110) mit einem Lanzettensitz (111) zur Montage einer Lanzette (10) an einem Ende;
einen Bedienmechanismus (120), der am anderen Ende des Lanzettenhalters (110) angebracht ist und der den Lanzettenhalter (110) entlang einem vorbestimmten Pfad lädt und loslässt;
ein Tiefeneinstellglied (140), das den Lanzettenhalter (110) aufnimmt und mindestens zwei Nuten (142) hat, die in einem vordefinierten Winkel an der Umfangsoberfläche eines Endes geformt sind, um im Uhrzeigersinn oder gegen den Uhrzeigersinn schräge Durchgänge (141, 141', 141") zu bilden; und
ein Gehäuse (130), das den Lanzettenhalter (110) und den Bedienmechanismus (120) aufnimmt, **dadurch gekennzeichnet, dass**
das Gehäuse (130) einen Vorsprung (131) hat, der an der Innenseite eines Endes angeformt ist und Schritt für Schritt in die Nuten (142) eingeführt wird, wenn das Tiefeneinstellglied (140) sich in einem vordefinierten Winkel dreht,
wobei das Gehäuse (130) weiter Folgendes einschließt:
ein erstes Gehäuseteil (132), das den Bedienmechanismus (120) aufnimmt; und
ein zweites Gehäuseteil (134), das sich um die Achse des ersten Gehäuseteils (132) dreht, kombiniert mit dem Tiefeneinstellglied (140) darin, und das den Vorsprung (131) hat;
die schrägen Durchgänge (141) und die Nuten (142) sind symmetrisch mit einer Lücke von 180 Grad geformt,
zwei Teile des Vorsprungs (131) sind symmetrisch mit einer Lücke von 180 Grad geformt, und die subkutane Eindringtiefe einer Lanzettennadel der Lanzette wird in zehn Schritten von 1,7 auf 2,0 mm verändert, und
über dem Vorsprung (131) ist ein Hohlraum (136) bestimmt.

2. Die Vorrichtung gemäß Anspruch 1, wobei mindestens zwei Führungsvorsprünge (135) in derselben Neigungsrichtung geformt sind wie der geneigte Durchgang (141), auf der Innenseite des zweiten Gehäuseteils (134), zur Führung der Kombination und Drehung zwischen dem zweiten Gehäuseteil (134) und dem Tiefeneinstellglied (140), und eine Rotationsführungsnut (143) im Tiefeneinstellglied entlang dem Bewegungspfad der Führungsvorsprünge (135) geformt ist.

3. Die Vorrichtung gemäß Anspruch 1, die weiter einen Deckel (150) umfasst, der aufklappbar am zweiten Gehäuseteil (135) angebracht ist und in der Mitte eine Durchgangsbohrung (151) hat, durch welche eine Lanzettennadel der Lanzette dringt.

4. Die Vorrichtung gemäß Anspruch 1, worin die schrägen Durchgänge (141) einen ersten schrägen Durchgang haben, bei dem die Neigung im Uhrzeigersinn von 0° auf 180° abnimmt, und einen zweiten schrägen Durchgang, bei dem die Neigung gegen den Uhrzeigersinn von 0° auf 180° zunimmt, wobei eine Stufe mit einer vorbestimmten Höhe an der Verbindungsstelle des ersten schrägen Durchgangs (141') und des zweiten schrägen Durchgangs (141") geformt ist.

5. Die Vorrichtung gemäß Anspruch 4, worin in jedem der ersten und zweiten schrägen Durchgänge (141', 141") zehn Nuten (142) geformt sind.

## Revendications

1. Dispositif à lancette avec une profondeur de pénétration hypodermique réglable, comprenant:
un porte-lancette (110) comportant un siège de lancette (111) pour monter une lancette (10) à une extrémité;
un mécanisme de commande (120) disposé à l'autre extrémité du porte-lancette (110) et chargeant et libérant le porte-lancette (110) le long d'un trajet prédéterminé,
un élément de réglage de profondeur (140) recevant le porte-lancette (110) et ayant au moins deux cannelures (142) formées à un angle prédéterminé sur la surface circonférentielle d'une extrémité pour former des passages inclinés (141,141', 141") dans le sens horaire ou anti-horaire; et
un boîtier (130) recevant le porte-lancette (110) et le mécanisme de commande (120), **caractérisé en ce que**
ledit boîtier (130) présente une saillie (131) qui est formée sur le côté intérieur d'une extrémité et est monté étape par étape dans les cannelures (142) lorsque l'élément de réglage de profondeur (140) tourne selon un angle prédéterminé,
le boîtier (130) incluant en outre
une première partie de boîtier (132) recevant le mécanisme de commande (120) ; et
une deuxième partie de boîtier (134) tournant autour de l'axe de ladite première partie de boîtier (132), en combinaison avec l'élément de réglage de profondeur (140) à l'intérieur de celle-ci, et présentant la saillie (131),
les passages inclinés (141) et les cannelures (142) sont formées symétriquement avec un espace de 180°,
deux pièces de la saillie (131) sont formées symétriquement avec un espace de 180° et la profondeur de pénétration hypodermique de l'aiguille de lancette de la lancette est réglée entre 1,7 et 2,00 mm en dix pas, et
un espace creux (136) est défini au dessus de la saillie (131).

2. Dispositif selon la revendication 1, dans lequel au moins deux saillies de guidage (135) sont formées dans la même direction d'inclinaison que le passage incliné (141), sur la face intérieure de la deuxième partie de boîtier (134), pour le guidage de la combinaison et de la rotation entre la deuxième partie de boîtier (134) et l'élément de réglage de profondeur (140), et une cannelure de guidage de rotation (143) est formée au niveau de l'élément de réglage de profondeur le long de la trajectoire de mouvement des saillies de guidage (135).

3. Dispositif selon la revendication 1, comprenant en outre un couvercle (150) articulé à la deuxième partie de boîtier (135) et ayant un trou débouchant (151) au centre à travers lequel passe une aiguille de lancette de la lancette.

4. Dispositif selon la revendication 1, dans lequel les passages inclinés (141) présentent un premier passage incliné avec une inclinaison décroissante dans le sens horaire de 0° à 180° et un deuxième passage incliné avec une inclinaison croissante dans le sens anti-horaire de 0° à 180°, et un gradin présentant une hauteur prédéterminée est formé au niveau de la jonction du premier passage incliné (141') et du deuxième passage incliné (141").

5. Dispositif selon la revendication 4, dans lequel dix cannelures (142) sont formées au niveau de chacun des premier et deuxième passages inclinés (141', 141").
